# EUROPEAN PATENT APPLICATION

(11) **EP 0 572 287 A2**
(43) Date of publication of application: **01.12.1993**
(21) Application number: 93400154.6
(22) Date of filing: 22.01.1993
(51) Int. Cl.: A61K 31/70

(54) **Use of cyclic double-stranded oligonucleotides containing the binding site for a transcription factor for inhibiting gene expression**

(30) Priority: 24.04.1992 WO PCT/FR92/00370
(71) Applicant: GENSET, F-75011 Paris (FR)
(72) Inventor: Blumenfeld, Marta, F-75013 Paris (FR); Clusel, Catherine, F-94290 Villeneuve le Roi (FR); Enjolras, Nathalie, F-75019 Paris (FR); Ugarte Romero, Edgarto, F-75019 Paris (FR); Vasseur, Marc, F-75005 Paris (FR)
(74) Representative: Warcoin, Jacques

(57) **Abstract**

The present invention concerns the use of a double-stranded closed oligonucleotide containing a binding site for a given transcription factor, e.g. HNF-1 factor, for inhibiting the expression of a given gene under the control of said transcription factor, e.g. liver-specific genes coding for fibrinogen, transthyretin, apolipoprotein B or the pre-S₁ gene of hepatitis B virus.

Particularly preferred oligonucleotides are the ones containing the sequence 5'GTTANT3', wherein N can be any of A,T,C or G.

## Description

### 1. FIELD OF THE INVENTION

The present invention concerns double-stranded circular or closed DNA oligonucleotides, a method for controlling the expression of genes with said circular oligonucleotides and pharmaceutical compositions comprising the same circular oligonucleotides. Circular oligonucleotides and their properties have been disclosed in the application FR n° 91 05114 and PCT/FR92/00370.

### 2. BACKGROUND OF THE INVENTION

Transcriptional control in eukaryotes results from the interplay between *cis*-acting DNA sequences in promoters, enhancers or silencers and *trans*-acting regulatory proteins (Maniatis et al., 1987; Jones et al., 1988; Johnson and McKnight, 1989; Mitchell and Tjian, 1989). Transcription initiation involves the establishment of an RNA polymerase II complex with several auxiliary factors at the TATA box (Breathnach and Chambon, 1981) or at the initiator element (Smale and Baltimore, 1989; Smale et al., 1990), to give the basal level of promoter activity (Saltzman and Weinmann, 1989; Sawadogo and Sentenac, 1990; Roeder, 1991). The activity of the basal transcriptional machinery is further modulated by the combined action of sequence-specific DNA binding factors (Johnson and McKnight, 1989; Mitchell and Tjian, 1989) and mediator or coactivator proteins, which may facilitate the formation of protein complexes between the DNA-binding proteins and the RNA polymerase II basal initiation machine. (Lewin, 1990; Ptashne and Gann, 1990; Pugh and Tjian, 1992).

Regulatory sequences and transcription factors have been identified by a combination of *in vitro* and *in vivo* methods. For example, *cis*-regulatory elements have been delineated in functional studies by stable or transient gene expression of cloned vectors in mammalian cells (Southern and Berg, 1982; Gorman et al., 1982; Selden et al., 1986; Gould and Subramani, 1988). Alternatively, cell-free extracts have been developed that allow accurate transcription from RNA polymerase II promoters using cloned DNA templates (Manley et al., 1980; Dignam et al., 1983; Gorski et al., 1986). Also, detection of low abondance DNA-binding activities in crude cell extracts has become possible due to sensitive DNA protection mapping (Galas and Schmitz, 1978) and gel retardation (Fried and Crothers, 1981; Garner and Revzin, 1981; Carthew et al., 1985) assays. Finally, purification of these proteins has been possible by the refinement of sequence-specific DNA affinity chromatography techniques (Rosenfeld and Kelly., 1986; Kadonaga and Tjian, 1986). The use of the above mentioned techniques has finally led to the characterization of both ubiquitous and tissue- or stage-specific transcription factors (reviewed in Locker and Buzard, 1990; Faisst and Meyer, 1992). While the former bind to regulatory sequences that control a variety of genes and are present in many different cell types, the latter are expressed only in a few cell types or are affected by various signal transduction pathways (muscle-specific, Olson, 1990; Weintraub et al., 1991; liver-specific, Mendel and Crabtree, 1991; Tronche and Yaniv, 1992; erythroid-specific, Rahuel et al., 1992; pituitary-specific, Ingraham et al., 1990; Schaufele et al., 1990).

Therefore, transcription factors (or trans-activators) are valuable targets for an oligonucleotide specific approach aiming at the control of gene expression.

### 3. SUMMARY OF THE INVENTION

The present invention provides a method for controlling gene expression in which double-stranded circular oligonucleotides are used to compete for the binding of protein factors to specific cellular or viral transcriptional or post-transcriptional control elements.

Another aspect of the present invention provides a double-stranded oligonucleotide in which both complementary strands are connected by loop domains to form a closed/circular oligonucleotide. The complementary strands comprise one or more than one binding site for said transcription factor and the closed/circular oligonucleotide can be used to inhibit the transcription of genes under the control of promoter/enhancer elements which bind to said transcription factor.

The present invention concerns double-stranded circular (closed) DNA oligonucleotides capable of binding a specific protein and thereby regulating RNA transcription involving specific DNA-protein interactions. Circular double-stranded oligonucleotides are resistant to exonucleolytic degradation. The methods of the invention are particularly useful in selectively inhibiting transcription of cellular and viral genes and oncogenes, and may be used in the treatment of a variety of diseases.

Circular double-stranded oligonucleotides containing an HNF-1 binding site can specifically inhibit transcription of liver-specific promoters.

More particularly, the present invention provides closed oligonucleotides containing an HNF-1 binding site and able to inhibit the expression of a given gene expressed under the control of this transcription factor. HNF-1 is a transcription factor which is involved in the control of various liver-specific genes of pathological interest. Among these genes, one can quote the genes coding for fibrinogen, transthyretin, apolipoprotein B, etc., as well as the pre-S1 gene of hepatitis B virus.

For example, the α and β fibrinogen promoters contain an HNF-1 site and the expression of these genes is strictly dependant of the presence of this factor (Courtois et al., 1987). It has been reported that increased level of fibrinogen is related to high risk of arterial thrombosis through an increase in blood and/or plasma viscosity (Lowe, 1992; Kelleher, 1992). Transthyretin, the serum carrier protein for thyroxine and vitamin A which is also regulated by HNF1 (Costa and Grayson, 1991), has been associated to amyloidosis with cardiac and neurologic involvement (Jacobson et al., 1992).Closed oligonucleotides containing the HNF-1 site, which down-regulate the expression of HNF-1 driven genes, are therefore anti-fibrinogen compounds which can be used in the treatment of coronary arterial diseases.

According to the most preferred embodiments of the invention, the circular double-stranded oligonucleotides can be composed of DNA, RNA or both. In addition, the nucleosides of the oligonucleotide can be joined by modified or unmodified phosphodiester linkages.

At last, the subject-matter of the present invention is the use of a compound as claimed in Claims 1 to 9.

When the transcription factor is HNF-1 factor, the said compound suitably consists of a double-stranded closed oligonucleotide containing at least the sequence 5'GTTANT 3', and its complementary strand, which are the half of the pseudo-palindromic HNF-1 binding sequence, wherein N is whatever nucleotide A, T, C or G.

In some embodiments, the said compound consists of a double-stranded closed oligonucleotide containing a binding site for the transcription factor HNF-1 selected among the followings :

1 5'GTTAATAATCTAC3'
3'CAATTATTAGATG5'

2 5'GTTACTAGTTAAC3'
3'CAATGATCAATTG5'

3 5'ATTAATAATTACA3'
3'TAATTATTAATGT5'

4 5'GTTAATATTCACC3'
3'CAATTATAAGTGG5'

5 5'GTTAATATTTAAT3'
3'CAATTATAAATTA5'

6 5'GTTAAAGTATAAC3'
3'CAAT TTCATATTG5'

7 5'GT TACTTAT TCTC3'
3'CAATGAATAAGAG5'

8 5'GTT TATTATTAGC3'
3'CAAATAATAATCG5'

9 5'GAAAATTATTTAC3'
3'CTT TTAATAAATG5'

1 0 5'ATTAATCATTACC3'
3'TAATTAGTAATGG5'

1 1 5'GTAATTGTTTACA3'
3'CATTAACAAATGT5'

1 2 5'GTTAATGTGTAAT3'
3'CAATTACACATTA5'

1 3 5'GTTAAAGTCAGTA3'
3'CAATTTCAGTCAT5'

1 4 5'GTTAATCATTACT3'
3'CAATTAGTAATGA5'

### 4. EXAMPLE 1: INHIBITION OF SPECIFIC DNA-PROTEIN INTERACTIONS BY CIRCULAR/CLOSED DOUBLE-STRANDED OLIGONUCLEOTIDES

### 4.1. MATERIALS AND METHODS

### 4.1.1. OLIGODEOXYNUCLEOTIDES

Oligodeoxynucleotides were synthesized using phosphoramidite chemistry. 5'phosphate oligonucleotides were synthesized using 5'Phosphate-On cyanoethyl phosphoramidite as the phosphorylating reagent.

Sequences of the oligodeoxynucleotides used in this study are depicted in Table I. The set of DNA oligodeoxynucleotides that was synthesized contains the binding site for the liver-specific transcription factor HNF-1 (Cereghini et al., 1988).

Concerning the set of HNF1 binding sites, PE56 double-stranded oligonucleotide contains the rat albumin sequence -63/-41 which encompasses the HNF1 binding site (Cereghini et al., 1988). DS34 contains four mutated bases which abolish completely HNF1 binding (Cereghini et al., 1988). GT-56ₒ and GT-56_{c}, and GT-34ₒ and GT-34_{c} are the corresponding non-ligated and ligated PE56 and DS34 dumbbell oligonucleotides.

### 4.1.2. SYNTHESIS OF LIGATED OLIGONUCLEOTIDE DUMBBELLS

Ligated oligodeoxynucleotide dummbells GT-56_{c} or GT-34_{c} (Table I) were obtained by ligation of the corresponding 5'phosphate oligonucleotides GT-56ₒ or GT-34ₒ with T4 DNA ligase. Standard ligation conditions were: 15 µM oligonucleotide, 50 mM Tris HCl pH 7.8, 10 mM MgCl₂, 20 µM DTT, 1 mM ATP, 1 mM BSA and 10.000 units/ml of T4 DNA ligase incubated in a total volume of 1 ml at 4°C for 48 hours. However, dumbbell ligation was also effective under the following conditions: 1-100 µM oligonucleotide, 5.000-100.000 units/ml of T4 DNA ligase, 4-37°C, 1-48 hours.

After ligation, reaction mixtures were concentrated by ethanol precipitation and ligated products were separated on a 12% polyacrylamide gel containing 7M urea. In each case, UV shadowing revealed a minor band comigrating with the GT-56ₒ or GT-34ₒ non-ligated oligonucleotide, along with a major new product which migrated more slowly than the respective non-ligated oligonucleotide. With other particular sequences, electrophoretical migration of the major ligation product may be similar, slower or faster than the corresponding non-ligated oligonucleotide.

The major ligation product was excised and eluted from the gel, precipitated with ethanol and quantitated by absorbance at 260 nm. In the case of ligation of oligonucleotides GT- 56ₒ or GT-34ₒ, the corresponding ligated dumbbells were obtained in 60 to 80% yields. The identity of the ligated dumbbells was confirmed by comigration with radioactive standards (see below).

Radioactive ligated dumbbells were obtained as follows. 5'phosphate GT-56ₒ or GT-34ₒ oligonucleotides were first incubated with calf intestine phosphatase under standard conditions and the dephosphorylated oligonucleotides were recovered by ethanol precipitation after phenol extraction. Two pmoles of the 5' OH oligonucleotide were phosphorylated with 30 µCi (γ ³²P)-ATP (3000 Ci/mmol) and T4 polynucleotide kinase, and then ligated with T4 DNA ligase and purified by denaturing polyacrylamide gel electrophoresis, as described for non-radioactive dumbbells, except that radioactive bands were detected by autoradiography and quantified by liquid scintillation counting. The identity of the ligated dumbbells was verified by incubation with alkaline phosphatase, phophodiesterase I or by S1 nuclease protection mapping (see below)

### 4.1.3. INCUBATION WITH ALKALINE PHOSPHATASE

Reaction mixtures contained 10 µM non-ligated or ligated radioactive oligonucleotide (10⁵ cpm/µg), 10 mM Tris HCl pH 8.0, 1 mM MgCl₂, 1 mM ZnCl₂, and 1 unit of calf intestinal alkaline phosphatase in a final volume of 10 µl. After incubation at 37°C for 30 min, the reaction was stopped by heating to 75°C for 10 min. After addition of 10 µl of formamide loading buffer, reaction products were analyzed on a 15% polyacrylamide gel containing 7 M urea. Radioactive bands were detected by autoradiography, and labeled products were quantified by excision from the gel and liquid scintillation counting.

### 4.1.4. INCUBATION WITH PHOSPHODIESTERASE I

Reaction mixtures contained 10 µM non-ligated or ligated radioactive oligonucleotide (10⁵ cpm/µg), 50 mM Tris HCl pH 7.5, 10 mM MgCl₂, 2 mM DTT and 5 x 10⁻⁵ units of phosphodiesterase I in a final volume of 10 µl. After incubation at 37°C for 30 min, the reaction was stopped by addition of 10 µl of formarmide loading buffer and reaction products were analyzed on a 15% polyacrylamide gel containing 7 M urea. Radioactive bands were detected by autoradiography, and labeled products were quantified by excision from the gel and liquid scintillation counting.

### 4.1.5. INCUBATION WITH S1 NUCLEASE

Reaction mixtures contained 10 µM non-ligated or ligated radioactive oligonucleotide (10⁵ cpm/µg), 50 mM NaCl, 33 mM sodium acetate pH 4.4, 30 µM ZnSO₄ and 1 unit of S1 nuclease in a final volume of 25 µl. After incubation at 37°C for 20 min, the reaction was stopped by addition of 20 mM EDTA, 0.3 M sodium acetate and 20 µg glycogen and oligonucleotides were precipitated with ethanol. After washing and drying, the DNA pellets were resuspended in 10 ml of formamide loading buffer and reaction products were analyzed on a 15% polyacrylamide gel containing 7 M urea. Radioactive bands were detected by autoradiography, and labeled products were quantified by excision from the gel and liquid scintillation counting.

### 4.1.6. PREPARATION OF LIVER NUCLEAR EXTRACTS

Nuclear extracts from rat liver were prepared as described in Cereghini et al., (1988). Protein concentration was determined by the BCA protein assay reagent (Pierce).

### 4.1.7. GEL RETARDATION ASSAYS

Binding reactions for band shift assays were performed in 14 µl of reaction mix containing: 10 mM Hepes pH 7.9, 50 mM KCl, 0.1 mM EDTA, 0.5 mM DTT, 10% (vol/vol) glycerol, 0.25 mM PMSF, 2.5 µg/ml aprotinin, 2.5 µg/ml leupeptin, 6 mM MgCl₂, 6 mM spermidine, 1.5 µg poly (dI-dC)·poly (dI-dC), 250 ng sonicated salmon sperm DNA, 3 fmol of (³²P)-labeled oligonucleotide probe and 0.2-5 µg of liver nuclear protein. Where indicated, specific or non-specific oligonucleotide competitors were added at the same time as the radioactive probe. After incubation at 4°C for 10 min, reaction mixtures were loaded onto a 6% polyacrylamide non-denaturing gel containing 0.25X TBE and were electrophoresed at 12 V/cm until a suitable separation was achieved. The gel was fixed, dried and exposed to X-ray film. Radioactive bands were quantitated by liquid scintillation counting.

### 4.2. RESULTS

### 4.2.1. ENZYMATIC CHARACTERIZATION OF CIRCULAR/CLOSED DUMBBELL OLIGONUCLEOTIDES

In many eukaryotic systems, antisense or sense oligonucleotides have been used to inhibit gene expression specifically. The application of oligonucleotides *in vivo* however, may be severely hampered by their sensitivity to nucleases that would make oligonucleotides unstable in a biological environment.

We were first concerned in verifying the identity of the closed dumbbell oligonucleotides by incubating them with alkaline phosphatase or with S1 nuclease.

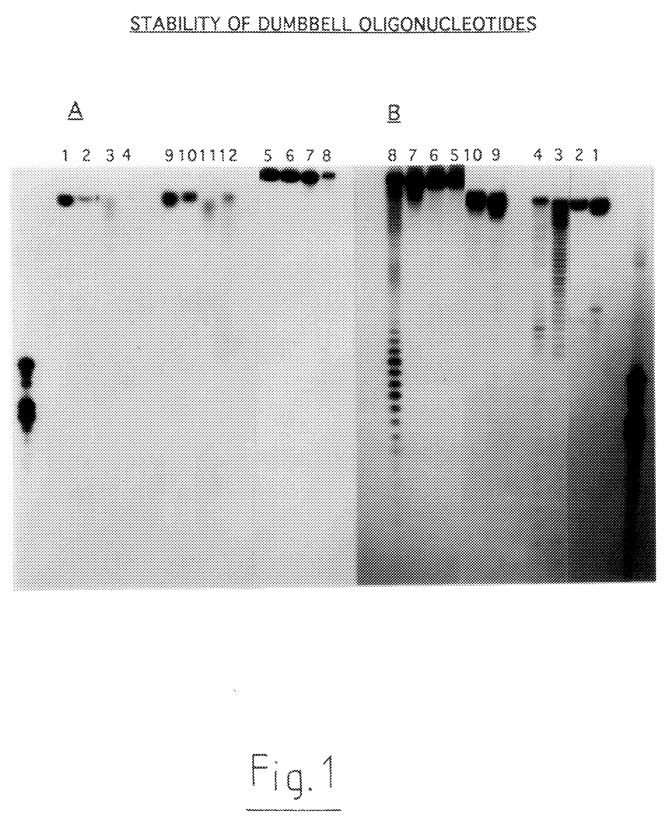

Figure 1A shows the results obtained with calf intestinal phosphatase. As expected for a closed molecule, the radioactive GT-56_{c} dumbbell was completely resistant to dephosphorylation (compare lanes 5 and 6), while under the same conditions the non ligated GT-56ₒ precursor almost completely lost the ³²P labeling (compare lanes 1 and 2). However, it is important to note that resistance to dephosphorylation by phosphatase cannot be used as the only criteria for characterizing a closed oligonucleotide, since protection of the 5'-³²P end might result from reasons other than oligonucleotide circularization. For example, the ligation reaction product that comigrates with the GT-56ₒ oligonucleotide and that is supposed to be the unreacted GT-56ₒ precursor, is resistant to dephosphorylation (Fig. 1A, compare lanes 9 and 10), while by 3' exonuclease degradation it can be shown that it is not a circular molecule (Fig.1A, compare lane 9 to lanes 11). The most possible explanation is that this 5' protected non-ligated dumbbell represents a 5' adenylylated reaction intermediate of the ligase reaction, as already reported for both T4 DNA and RNA ligases (Cranston et al., 1974; Sugino et al., 1977).

We further confirmed the structure of GT-56ₒ and GT-56_{c} by incubating them with S1 nuclease. This enzyme will attack the single-stranded regions of the dumbbells (the interstrand connecting loops), thus allowing to recover the double-stranded portion of the oligonucleotide. In the case of the non-ligated dumbbell GT-56ₒ, the radioactive label should be associated to a 5' end-labeled 12-mer, while for the ligated dumbbell GT-56_{c} the radioactive strand should be a 23-mer inner-labeled oligonucleotide (see Table I). As shown in Figure 1B, a 23-mer protected fragment was obtained for GT-56_{c} (lane 8), clearly indicating that GT-56_{c} corresponds to a ligated dumbbell in which the complementary sequences are mostly in a double-stranded structure. On the contrary, no 12-mer radioactive fragment was observed in the case of the non-ligated precursor GT-56ₒ (Fig. 1B, lane 4); instead, most of the label was associated to small size oligomers (<6 nucleotides). This result might indicate that the single-stranded form of the complementary region in the non-ligated dumbbell is more stable than the double-stranded structure; thus, S1 nuclease cleavage of the partially deshybridized duplex would result in a rapid lose of the 5'-(³²P) label.

It has recently been reported that degradation of unmodified phosphodiester oligodeoxynucleotides in cultured cells, culture media and serum, takes place primarily as a result of 3'exonuclease activity (Wickstrom, 1986; Tidd and Varenius, 1989; Eder et al., 1991; M. Blumenfeld et al. unpublished results). The serum 3' exonuclease shows a strong preference, if not an absolute requirement, for single-stranded substrates and has no associated 5' exonuclease or endonuclease activities, but may have a phosphatase associated activity (Eder et al., 1991; M. Blumenfeld et al. unpublished results). To overcome the problem of losing the 5' label in the non-ligated dumbbell by dephosphorylation in serum, we compared the stability of the open or closed dumbbells to 3' exonucleolytic degradation by incubating them with phosphodiesterase I, a well-characterized 3' exonuclease from snake venom. As shown in Figure 1 A and B, the ligated dumbbell GT-56_{c} was mostly resistant to degradation (compare lanes 5 to 7), while under the same conditions the open dumbbell precursor GT-56ₒ was almost completely degraded by PDEI (compare lanes 1 to 3).

In conclusion, the enzymatic studies presented above indicate that the closed dumbbells described in the present invention preferentially adopt a double-stranded structure along the whole complementary stem region. The double-stranded closed structure should efficiently protect the dumbbell oligonucleotides from 3' exonucleases which are largely responsible for degradation of oligonucleotides *in vivo* as well as in serum and cell extracts (Zendegui et al., 1992; Wickstrom, 1986; Tidd and Varenius, 1989; Eder et al., 1991). Even in the case of a minor degradation by a cellular single-stranded endonuclease, this would result in the generation of a stable and still functional double-stranded oligonucleotide, thus allowing to consider the closed dumbbell oligonucleotide described in the present invention as a prodrug compound.

### 4.2.2. SPECIFIC BINDING OF THE LIVER TRANSCRIPTION FACTOR HNF-1 TO A CLOSED DUMBBELL CARRYING THE HNF-1 TARGET SITE

In order to consider the possible therapeutical application of the dumbbell oligonucleotides described in the present invention as tools for controlling particular gene products, it is critical to first examine the ability of the targeted DNA-binding protein to discriminate between specific and non-specific dumbbells in an *in vitro* binding system. The gel mobility shift assay (Fried and Crothers, 1981; Garner and Revzin, 1981) provides such an *in vitro* test, since it is an extremely sensitive method which allows to detect and characterize specific DNA-protein interactions, even when working with crude protein preparations, such as whole cell or nuclear extracts.

On the other hand, we chose liver nuclear extracts since they are the source of well studied DNA-binding factors, such as HNF-1 (Cereghini et al., 1988; Hardon et al., 1988; Courtois et al., 1988) among others, which can also be tested for transcriptional activity both *in vitro* and *in vivo* .

We have first compared the relative binding affinity of rat liver HNF-1 towards an HNF-1 site contained in a double stranded oligonucleotide or in an open dumbbell oligonucleotide or in a closed dumbbell oligonucleotide (Table I, dsPE56, GT-56ₒ or GT-56_{c}, respectively). This particular HNF-1 binding site corresponds to the sequence present in the rat albumin promoter, which has been shown to be a high affinity site when compared to other natural HNF-1 targets or to mutated sites (Cereghini et al., 1988).

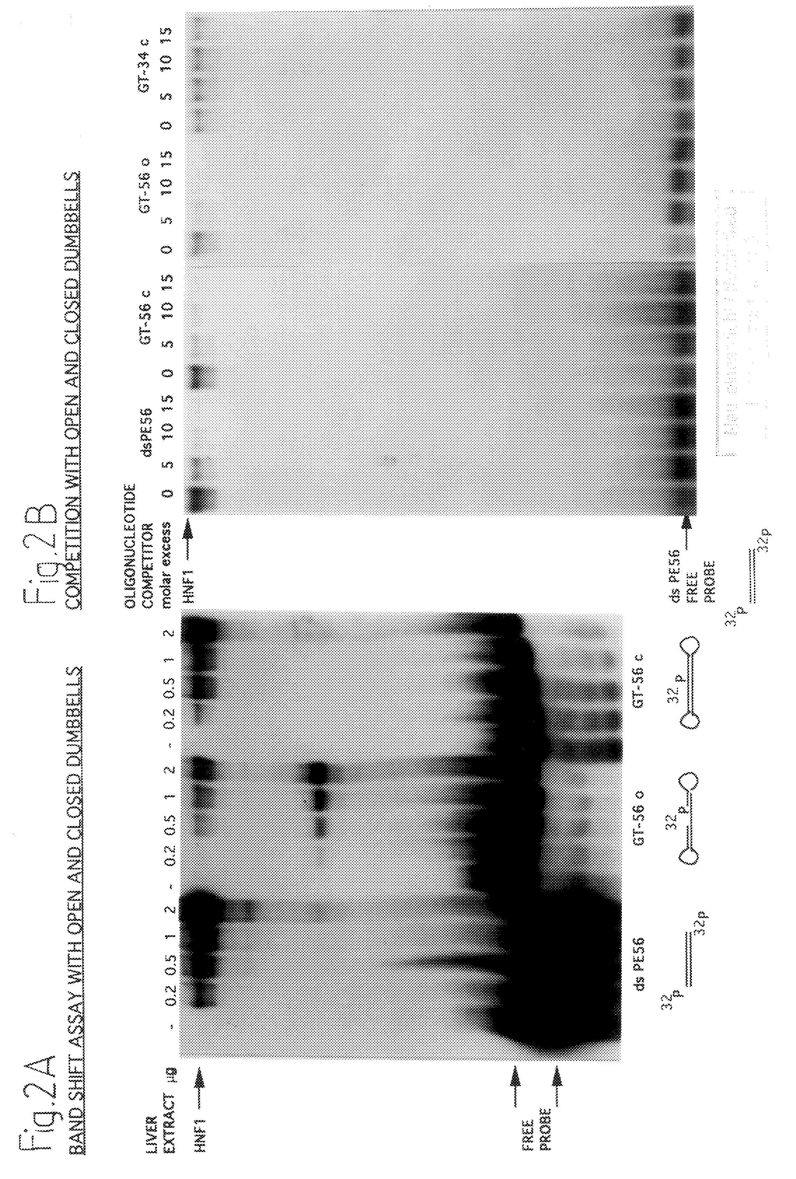

As shown in Figure 2-A, when we incubated radioactive dsPE56, GT-56ₒ or GT-56_{c} probes with increasing amounts of liver nuclear proteins, a unique DNA-protein complex corresponding to the HNF-1 retarded band was readily observed in the case of dsPE56 and GT-56_{c}, respectively. On the contrary, the results with the non-ligated dumbbell probe GT-56ₒ were quite different, since two DNA-protein complexes were detected: a minor one migrating at the HNF-1 position and a major band migrating faster. The latter band might correspond to a single-stranded non-specific DNA-binding protein, since it comigrates with a complex observed upon incubation of liver extracts with some single-stranded probes (M. Blumenfeld et al., unpublished results).

Quantitative analysis, taking into acount the specific activity of each probe, revealed that HNF-1 binds to a closed dumbbell carrying its specific recognition sequence with the same relative affinity as to a double-stranded target site. In contrast, a five-fold decrease of HNF-1 retarded band was observed with the non-ligated dumbbell probe, GT-56ₒ, together with the formation of a different major complex. Taken together, these data may indicate that the stability of the double-stranded structure in the non-ligated dumbbell is lower than that of the ligated oligonucleotide, since it can be more easily displaced to a single-stranded available form. The same conclusion could be drawn from the enzymatic structural analysis discussed in Section 4.2.1.

As shown in Figure 2-B, similar results were obtained by comparing the relative binding efficiencies of the different oligonucleotides in competition experiments. Again, as for the direct gel retardation assay using each oligonucleotide as a probe, addition of unlabeled GT-56_{c} or ds PE56 competed for HNF-1 binding to radioactive ds PE56 in the same way, while unlabeled GT-56ₒ was about five-fold less effective competitor.

Finally, in order to assess the specificity of the binding to a dumbbell oligonucleotide, we synthesized a closed dumbbell that contains an HNF-1 mutated site, GT-34c (Table I), that totally abolishes HNF-1 binding (Cereghini et al., 1988). The mutated closed dumbbell was then analyzed in a competition experiment. Addition of a 15-fold molar excess (Figure 2-B) or even a 600-fold excess (not shown) of cold GT-34c did not compete with labeled PE56 for HNF-1 binding; under the same conditions, GT-56_{c} or ds PE56 almost completely inhibited the binding of the radioactive probe.

The results presented here indicate that the presence of interstrand connecting loops in a phosphodiester dumbbell oligonucleotide does not impose a physical constraint to a DNA-protein interaction in terms of changing the affinity or the specificity of the binding. Since in addition, the closed dumbbell oligonucleotides described in the present invention are more efficiently protected from exonucleolytic degradation than their non-ligated counterparts (Section 4.2.1), these molecules have a great potential to be used as therapeutic agents.

### 5. EXAMPLE 2: IN VITRO TRANSCRIPTION

### 5.1 MATERIALS AND METHODS

### 5.1.1.PLASMID CONSTRUCTIONS

pALB-GF contains the rat albumin promoter cloned in front of a 375 bp G-less cassette. The albumin sequence was obtained by ligation of six overlapping double-stranded oligonucleotides as described in Tronche et al., 1989, except that the TF6/TF7 oligonucleotides were replaced by: 
5' G T T A G A G A A G T A T A T T A G A G C G A G T G 3'
3' TC TC T TCATATAATCT CGCTCACTTAA 5'
thus generating a -152/-14 rat albumin sequence flanked by BglII and EcoRI 5'and 3'cohesive ends, respectively. This promoter was cloned between the BglII and EcoRI sites from p(C₂AT)₁₉BE, obtained by inserting a polylinker containing unique BglII, XbaI, XhoI and BamHI restriction sites in the EcoRI site from p(C₂AT)₁₉ (Sawadogo and Roeder, 1985). Since the inserted promoter lacks the transcription start site, initiation takes place in the vector sequences giving a transcript of about 370 nt.

pML(C₂AT)₁₉ (Sawadogo and Roeder, 1985) contains the adenovirus 2 major late promoter (-400/+10) linked to a 375 bp G-less cassette, generating a 385 nt transcript. In pAlb400 (Gorski et al.), the mouse albumin promoter (-650/+22) directs the transcription of a 398 nt G-free transcript.

### 5.1.2. IN VITRO TRANSCRIPTION ASSAY

Transcription assays were performed using the "G-free cassette" system developed by Sawadogo and Roeder (1985 ). Reactions were carried out in a volume of 20 µl of 25 mM Hepes pH 7.6, 50 mM KCl, 10% glycerol, 6 mM MgCl₂, 0.6 mM ATP and CTP, 0.1 mM 3-O-methyl-GTP, 7 µCi (α-³²P)-UTP (3000 Ci/mmol), 5 µM UTP, 6 nM of pAlb400 or pAlbGF specific template DNA, 2 nM of pML(C₂AT)₁₉ control DNA, 30 units of RNasin. and 1.6 mg/ml of liver nuclear protein. Transcription efficiency was optimized by varying the relative amounts of DNA template and nuclear extract.

DNA templates were preincubated with extracts for 10 min on ice, and transcriptions were initiated by adding the nucleotides. After incubation at 30°C for 1 h, the reactions were stopped by addition of 280 µl of 20 mM Tris pH 7.5, 0.25 M NaCl, 1% SDS, 5 mM EDTA, followed by digestion with 40 µg of proteinase K for 30 min at 37°C. The transcription products were then extracted with phenol, precipitated with ethanol and analyzed by electrophoresis on a 4% acrylamide/ 7 M urea gel. Gels were dried and exposed to X-ray films at -80°C with intensifying screen.

### 5.2. RESULTS

*In vitro* reactions containing extracts of mammalian cells or tissues (Manley et al.,1980; Dignam et al., 1983; Gorski et al., 1986) have been shown to yield accurate transcription initiation by RNA pol II. An *in vitro* transcription assay can then be used for testing the ability of dumbbell oligonucleotides to interfere with the transcription of a specific gene.

HNF-1 is a homeodomain-containing transcriptional regulator that is essential for the liver-specific expression of albumin and many other hepatic genes (reviewed in Tronche and Yaniv, 1992). *In vitro* studies confirmed the crucial role of HNF-1 in hepatocyte-specific transcription: mutation of the HNF-1 binding site drastically reduces the transcription of the albumin promoter (M. B., unpublished results; Maire et al., 1989) and, in addition, purified HNF-1 is sufficient to complement a spleen nuclear extract for the *in vitro* transcription of the mouse albumin promoter (Lichsteiner and Schibler, 1989). The *in vitro* transcription of the albumin promoter is then a suitable model for studying the effect of HNF-1 dumbbells as specific inhibitors of transcription.

In order to allow an easy and rapid analysis of the *in vitro* synthesized transcripts, we used the assay system developed by Sawadogo and Roeder (1985), in which the promoter is linked to an artificial DNA fragment that does not contain G residues on the transcribed strand (G-free cassette). In the absence of GTP, the RNA polymerase will only synthesize RNA on the "G-free cassette", the size of the transcript being defined only by the cap site and the first G residue flanking the 3' border of the cassette. This extremely sensitive assay has the technical advantage of allowing the use of supercoiled covalently closed circular DNA templates, which are more efficiently transcribed (Shapiro et al., 1988), together with the direct labeling of the transcripts.

We used two different albumin-specific plasmids: pAlb400, which contains the mouse albumin promoter and pALB-GF, which contains the rat albumin promoter (see Section 5.1.1.). The pAlb400 mouse albumin construct contains the promoter sequences from position -650 to +22 and gives a G-less transcript of about 400 nucleotides; an equivalent construction from the rat albumin promoter cannot be used in this assay because there is a G-residue at the +14 position. In addition, transient expression assays indicated that the first 150 bp of the rat albumin 5'- flanking region are necessary and sufficient for tissue- or cell-specificity (Heard et al., 1987). Therefore, the rat albumin sequences used for the *in vitro* transcription studies corresponded to the fragment -152 to -14 from the promoter. Since the inserted promoter lacks the transcription start site, initiation takes place in the vector sequences giving a transcript of about 370 nt.

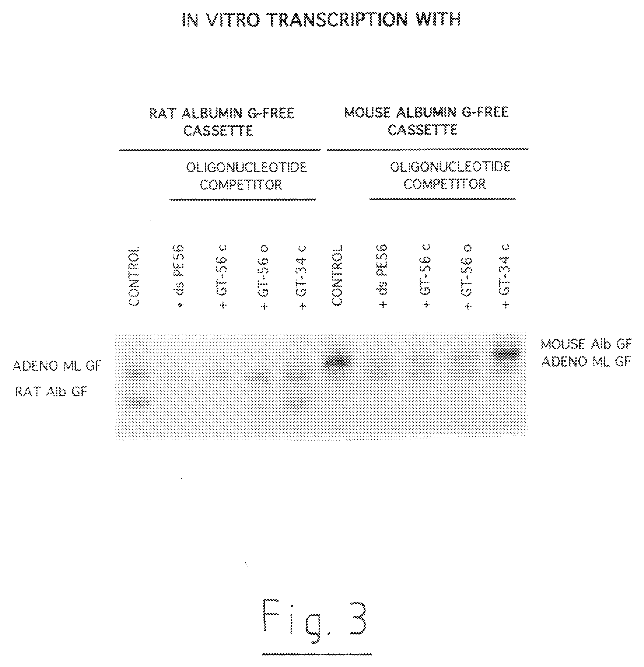

As shown in Figure 3, when liver nuclear extracts were used to transcribed 6 nM of pAlb400 in the presence of 2 nM of pML(C₂AT)₁₉, the albumin-specific transcript was transcribed about 10 times more than the adenovirus major late promoter control. On the contrary, when transcription was carried out in the presence of 60 nM of dsPE56 or GT-56_{c}, the albumin-specific transcription was reduced to 10%, while the adenovirus control was not affected. It is interesting to note that transcription of a template containing the DS34 mutation in the HNF-1 site also gives 10% of the wild type template transcription (M.B, unpublished results), thus indicating that a 10-fold molar exces of GT-56_{c} is able to quantitatively compete for all the HNF1-dependent transcription. Most important, the specificity of the HNF1-dumbbell interaction was not modified in the *in vitro* transcription system, since a 10-fold (Fig. 3) or a 50-fold molar exces (not shown) of GT-34c HNF-1 mutant dumbbell did not affect the albumin-specific signal. Concerning GT-56ₒ, the *in vitro* transcription competition correlated quite well with the binding experiments: GT-56ₒ was about 2 times less effective competitor than GT-56_{c} (Fig.3), while its binding relative affinity was about 5 times less than GT-56_{c} (Fig.2; see Section 7.2.2.).

Essentially similar results were obtained when the rat albumin promoter was used (Fig. 3).

Double-stranded unmodified oligonucleotides containing binding sequences for HNF1 (Cereghini et al., 1988), Sp1 (Wu et al., 1990) or BPV-1 E2 transactivator (Dostatni et al., 1991) have been shown to effectively inhibit transcription in eukaryotic *in vitro* systems. The presented results indicate that the stable dumbbell oligonucleotides described in the present invention can be succesfully used as competitors for the normal assembly of the transcriptional machinery, thus resulting in inhibition or activation of a particular gene.

### 6 .EXAMPLE 3: EX VIVO INHIBITION IN A TRANSIENT EXPRESSION ASSAY

### 6.1. MATERIALS AND METHODS

### 6.1.1. PLASMID CONSTRUCTIONS

pΔE1 Alb-CAT (Heard et al.) contains CAT under the control of the rat albumin promoter (-151/+16). pSVe-CAT has been described in Ott et al (1984). pRSV-HNF1 contains the cDNA for the rat transcription factor HNF1 under the control of the RSV LTR (Chouard et al., 1990)

### 6.1.2. DNA TRANSFECTION AND TRANSIENT EXPRESSION ASSAYS

C33 human epithelial tumour cells (Yee et al., 1985) were cultured in DMEM supplemented with 10% fetal calf serum and were transfected by the standard calcium phosphate coprecipitation protocol (Ausubel et al., 1989).

Falcon dishes (6 cm) containing 5 x 105 C33 cells, were transfected in triplicates with a mixture of pΔE1 Alb-CAT (2 µg) and pRSV-HNF1 (0.5 µg) or with 1 µg of SVeCAT; the total amount of DNA transfected was mantained constant (5 mg) by addition of a RSV-luciferase plasmid. Where indicated, oligonucleotides were included in the calcium phosphate-DNA precipitate. The precipitate was left on the cells overnight, and cell extracts were prepared 48 hours after removal of the precipitate. CAT activity was measured by the phase-extraction assay using ¹⁴C-chloramphenicol and butyryl CoA as substrates (Ausubel et al., 1989). Values lower than twice the background obtained in non transfected cells were discarded.

### 6.2. RESULTS

We were finally interested in testing the ability of the dumbbell oligonucleotides described in the present invention to regulate the gene expression in an *ex vivo* cellular system. For doing so, we used a transient transfection expression assay which allowed us to control the intracellular level of the target protein HNF1.

C33 human epithelial tumour cells do not express the HNF-1 protein; therefore, when they are transfected with a CAT vector controlled by the HNF-1 dependent albumin promoter there is no detectable CAT activity. However, when C33 cells are cotransfected with an HNF-1 expression vector, they express the CAT protein proportionally to the quantity of HNF1 expression vector added (Rey-Campos et al., 1991).

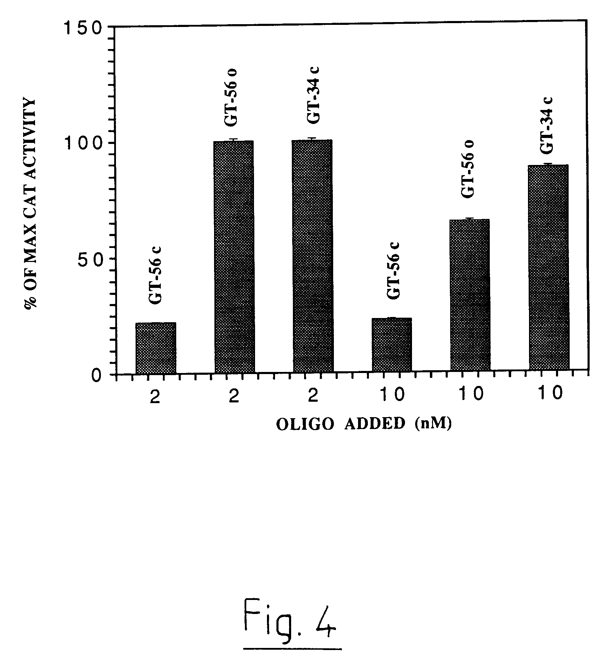

In order to better evaluate the competition efficiency of the dumbbell oligonucleotides, we set up the conditions for cotransfecting C33 cells with subsaturating amounts of RSV-HNF1 expression vector, and we then cotransfected different amounts of GT-56ₒ, GT-56_{c} or GT-34_{c} oligonucleotides. As shown in Figure 4, 2 nM GT-56_{c} inhibited almost 80% of the HNF-1 specific transcription, while under the same conditions GT-56ₒ or GT-34_{c} were ineffective. At higher concentrations (Fig.4, 10 nM), GT-56_{c} specific inhibition was still substantial, but was somehow overlapped by a slight non-specific inhibition (Fig.4, 10-30%). It is interesting to note that GT-56ₒ was unable to inhibit in the transient expression system, while there was only a twofold difference in the inhibition properties of GT-56ₒ and GT-56_{c} in the *in vitro* transcription assay (Fig.3 and Section 7.2). This results might reflect the more stable conformation of GT-56_{c} in the *in vivo* experimental conditions and are in agreement with the results presented in the previous Sections.

Gilman and coworkers reported that microinjected unmodified double-stranded c-fos CRE or AP1 binding sites are able to block c-fos induction by extracellular stimuli or to block initiation of DNA sytnthesis, respectively, probably by competing for CREB or JUN binding (Berkowitz et al., 1989; Riabowol et al., 1992). Since the authors used a microinjection procedure, it is difficult to evaluate the concentration effects of the oligonucleotides and their relative stability in the culture medium. Using a transfection approach similar to that described in this section, Bielinska et al. (1990) reported efficient inhibition by octamer or NF-kB unmodified or phosphorothioate double-stranded oligonucleotides at the mM range of concentration.; however, non-specific inhibition at these concentration was about 30%. The results presented in this section show that the use of nanomolar concentrations of non-modified dumbbell oligonucleotides results in highly efficient specific inhibition of gene expression.

TABLE I. OLIGONUCLEOTIDES USED

dsPE56 -63 T G T G G T T A A T G A T C T A C A G T T A
C A C C A A T T A C T A G A T G T C A A T A -41

T T T T
GT 560 T G T G G T T A A T G pA T C T A C A G T T A T
T T
T A C A C C A A T T A C T A G A T G T C A A T A
T T T T

T T T T
GT 56c T G T G G T T A A T G A T C T A C A G T T A T
T T
T A C A C C A A T T A C T A G A T G T C A A T A
T T T T

T T T T
GT 34c T G T G G T G T A T G A G G T A C A G T T A T
T T
T A C A C C A C A T A C T C C A T G T C A A T A
T T T T

### BRIEF DESCRIPTION OF THE FIGURES

Figure 1. Stability of dumbbell oligonucleotides. Fig.1A shows a short time exposure autoradiograph of the products obtained by incubation of GT-56ₒ (lanes 1 to 4) or GT-56_{c} (lanes 5 to 8) with different enzymes. Lanes 9 to 12 show the enzymatic characterization of the oligonucleotide comigrating with GT-56ₒ after ligation. Lanes 1, 5 and 9 untreated oligonucleotides; lanes 2, 6 and 8, incubation with calf intestinal alkaline phosphatase; lanes 3, 7 and 9, incubation with snake venom phosphodiesterase 1; lanes 4, 8 and 12, incubation with S1 nuclease. Fig. 1B depicts a longer exposed autoradiograph.

Figure 2. Band shift assays and competitions with open and closed HNF1 dumbbells. Fig 2A depicts the autoradiograph of a gel retardation assay using increasing amounts of liver nuclear extracts and HNF1 probes in the form of double-stranded oligonucleotide dsPE56, open dumbbell GT-56ₒ or closed dumbbell GT-56_{c}, as indicated. The arrows indicate the positions of the HNF1 specific complex and the free probes, respectively. Fig.2B shows the autoradiograph of a gel retardation assay carried out with 1 µg of liver nuclear extract and 3 fmoles of ³²P dsPE56 in the presence of the indicated amounts of unlabeled dsPE56, GT-56_{c}, GT-56ₒ or GT-34_{c} oligonucleotides. Lanes designed 0 contained no competing oligonucleotides.

Figure 3. Competition of open and closed HNF1 dumbbells in an *in vitro* transcription assay. The autoradiograph shows a transcription assay using liver nuclear extracts with 2 nM of pML(C₂AT)₁₉ internal control template (adenovirus major late promoter G-free cassette) and 6 nM of pALB-GF (rat albumin G-free cassette) or 6 nM of pAlb400 (mouse albumin G-free cassette), in the presence of 60 nM dsPE56, GT-56_{c}, GT-56ₒ or GT-34_{c} dumbbells, as indicated. Lanes designed control contained no competing oligonucleotides. ADENO ML GF indicates the adenovirus major late promoter transcript (385 nt); MOUSE Alb GF indicates the 400 nt transcript from the mouse albumin promoter; RAT Alb GF indicates the 370 nt transcript originated from the rat albumin promoter.

Figure 4. Inhibition of HNF1 transcriptional activity in C33 cells by HNF1 dumbbells. GT-56_{c}, GT-56ₒ or GT-34_{c} dumbbells were cotransfected in C33 cells with pΔE1 Alb-CAT reporter vector and pRSV-HNF1 expression vector at the indicated concentrations. 100% refers to the maximal CAT activity obtained at 2nM GT-56ₒ or GT-34_{c}.

### BIBLIOGRAPHY

Ausubel, F.M., Brent, R., Kingston, R.E., Moore, D.D., Seidman, J.G., Smith, J.A. and Struhl, K. (1989) *Current Protocols in Molecular Biology*. Greene Publishing Associates and Wiley - Interscience, New York.

Berkowitz, L.A., Riabowol, K.T. and Gilman, M.Z. (1989). *Mol. Cell. Biol*. **9**, 4272-4281.

Bielinska, A., Shivdasani, R.A., Zhang, L. and Nabel, G.J. (1990). *Science* **250**, 997-1000.

Breathnach, R. and Chambon, P. (1981). *Annu. Rev. Biochem.* **50**, 349-383.

Carthew, R.W., Chodosh, L.A. and Sharp, P.A. (1985). *Cell* **43**, 439-448.

Cereghini, S., Blumenfeld, M. and Yaniv, M. (1988) *Genes and Development*, **2**, 957-974.

Chouard, T., Blumenfeld, M., Bach, I., Vandekerckhove, J., Cereghini, S. and Yaniv, M. (1990) *N.A.R.*, **18**, 5853-5863.

Costa, R.H. and Grayson, D.R. (1991). *Nucleic Acids Res.* **19**, 4139-4145.

Courtois, G., Baumhueter, S. and Crabtree, G.R. (1988). *Proc. Natl.* *Acad.* *Sci. USA* **85**, 7937-7941.

Courtois, G., Morgan G.J., Campbell, L.A., Fourel, G. and Crabtree, G.R. (1987). *Science* **238**, 688-692.

Cranston, J.W., Silber, R., Malathi, V.G. and Hurwitz, J. (1974). *J.* *Biol.* *Chem.* **249**, 7447-7456.

Dignam, J.D., Lebovitz, R.M. and Roeder, R.G. (1983). *Nucleic Acids Res.* **11**, 1475-1489.

Dostatni, N., Lambert, P.F., Sousa, R., Ham, J., Howley, P.M. and Yaniv, M (1991). *Genes Dev.* **5**, 1657-1671.

Eder, P.S., DeVine, R.J., Dagle, J.M. and Walder, J.A. (1991). *Antisense* *Res. Dev.* **1**, 141-151.

Faisst, S. and Meyer, S. (1992). *Nucleic Acids Res.* **20**, 3-26.

Fried, M. and Crothers, D.M. (1981). *Nucleic Acids Res.* **9**, 6505-6525.

Galas, D.J. and Schmitz, A. (1978). *Nucleic Acids Res.* **5**, 3157-3170.

Garner, M.M. and Revzin, A. (1981). *Nucleic Acids Res.* **9**, 3047-3060.

Gorman, C.M., Moffat, L.F. and Howard, B.H. (1982). *Mol. Cell. Biol.* **2**, 1044-1051.

Gorski, K., Carneiro, M. and Schibler, U. (1986). *Cell* **47**, 767-776.

Gould, S.J. and Subramani, S. (1988). *Anal. Biochem.* **7**, 5-13.

Hardon, E.M., Frain, M., Paonessa, G. and Cortese, R. (1988). *EMBO J.* **7**, 1711-1719.

Heard, J.-M., Herbomel, P., Ott, M.-O., Mottura-Rollier, A., Weiss, M. and Yaniv, M. (1987). *Mol. Cell. Biol.* **7**, 2425-2434.

Ingraham, H.A., Flynn, S.E., Voss, J.W., Albert, V.R., Kapiloff, M.S., Wilson, L. and Rosenfeld, M.G. (1990). *Cell* **61**, 1021-1033.

Jacobson, D.R., McFarlin, D.E., Kane, I. and Buxbaum, J.N. (1992). *Hum. Genet.* **89**, 353-356.

Johnson, P.F. and McKnight, S.L. (1989). *Annu. Rev. Biochem.* **58**, 799-839.

Jones, N.C., Rigby, P.W.J. and Ziff, E.B. (1988). *Genes Dev.* **2**, 267-281.

Kadonaga, J.T. and Tjian, R. (1986). *Proc. Natl. Acad. Sci. USA* **83**, 5889-5893.

Kelleher, C. C. (1992). *Eur. J. Epidemiol.* **8**, 79-82.

Lewin, B. (1990). *Cell* **61**, 1161-1164.

Lichsteiner, S. and Schibler, U. (1989). *Cell* **57**, 1179-1187.

Locker, J. and Buzard, G. (1990). *DNA Sequence-J.DNA Sequencing and Mapping* **1**, 3-11.

Lowe, G.D.O. (1992). *Thromb. Haemostas.* **67**, 494-498.

Maire, P., Wuarin, J. and Schibler, U. (1989) *Science* **244**, 343-346.

Maniatis, T., Goodbourn, S. and Fischer, J.A. (1987). *Science* **236**, 1237-1245.

Manley, J.L., Fire, A., Cano, A., Sharp, P.A. and Gefter, M.L. (1980). *Proc.* *Natl. Acad. Sci. USA* **77**, 3855-3859.

Mendel, D.B. and Crabtree, G.R. (1991).*J. Biol. Chem.* **266**, 677-680.

Mitchell, P.J. and Tjian, R. (1989) *Science* **245**, 371-378.

Olson, E.N. (1990). *Genes and Dev.* **4**, 1454-1461.

Ott, M.O., Sperling, L., Herbomel, P., Yaniv, M. and Weiss, M. (1984) *EMBO* *J.*, **3**, 2505-2510.

Ptashne, M. and Gann, A.A.F. (1990). *Nature* **346**, 329-331.

Pugh, B. F. and Tjian, R. (1992). *J. Biol. Chem.* **267**, 679-682.

Rahuel, C., Vinit, M.-A., Lemarchandel, V., Cartron, J.-P. and Roméo, P.-H (1992). *EMBO J.* **11**, 4095-4102.

Rey-Campos, J., Chouard, T., Yaniv, M. and Cereghini, S. (1991). *EMBO J.* **10**, 1445-1457.

Riabowol, K., Schiff, J. and Gilman, M.Z. (1992). *Proc. Natl. Acad. Sci.USA* **89**,, 157-161.

Roeder, R. (1991). *Trends Biochem. Sci.* **16**, 402-408.

Rosenfeld, P.J. and Kelly, T.J. (1986). *J. Biol. Chem.* **261**, 1398-1408.

Saltzman, A.G. and Weinmann, R. (1989) *FASEB. J.***3**, 1723-1733.

Sawadogo, M. and Roeder, R. (1985) *Proc.Natl.Acad.Sci.* , **82**, 4394-4398.

Sawadogo, M. and Sentenac, A. (1990). *Annu. Rev. Biochem.* **59**, 711-754.

Schaufele, F., West, B.L. and Reudelhuber, T. (1990). *Nucleic Acids Res.* **18**, 5235-5244.

Selden, R.F., Burke-Howie, K., Rowe, M.E., Goodman, H.M. and Moore, D.D. (1986). *Mol. Cell. Biol.* **6**, 3173-3179.

Shapiro, D.J., Sharp, P.A., Wahli, W.W. and Keller, M.J. (1988). *DNA* **7**, 47-55.

Smale, S.T. and Baltimore, D. (1989). *Cell* **57**, 103-113.

Smale, S.T., Schmidt, M.C., Berk, A.J. and Baltimore, D. (1990). *Proc.* *Acad. Natl. Sci.. USA* **87**, 4509-4513.

Southern, P.J. and Berg, P. (1982). *J. Mol. Appl. Gen.* **1**, 327-341.

Sugino, A., Snopek, T.J. and Cozzarelli, N.R. (1977). *J. Biol. Chem.* **252**, 1732-1738.

Tidd, D.M. and Varenius, H.M. (1989). *Br. J. Cancer* **60**, 343-350.

Tronche, F. and Yaniv, m. (1992). *BioEssays* **14**, 1-9.

Tronche, F., Rollier, A., Bach, I., Weiss, M.C. and Yaniv, M. (1989) *Mol. Cell.Biol.*, **9**, 4759-4766.

Weintraub, H., Davis, R., Tapscott, S., Thayer, M., Krause, M., Benezra, R., Blackwell, T., Turner, D., Rupp, R., Hollenberg, S., Zhuang, Y. and Lassar, A. (1991). *Science* **251**, 761-766.

Wickstrom, E. (1986). *J. Biochem. Biophys. Methods* **13**, 97-102.

Wu, H., Holcenberg, J.S., Tomich, J., Chen, J., Jones, P.A., Huang, S.-H. and Calame, K. L. (1990). *Gene* **89**, 203-209.

Yee, D., Krishnan-Hewlett, I., Baker, C.C., Schegel, R. and Howley, P.M. (1985) *Am.J. Pathol.*, **119**, 361-366.

Zendegui, J.G., Vasquez, K.M., Tinsley, J.H., Kessler, D.J. and Hogan, M.E. (1992). *Nucleic Acids Res.* **20**, 307-314.

## Claims

**1-**Use of a compound consisting of a double-stranded closed oligonucleotide containing a binding site for a given transcription factor, for inhibiting the expression of a given gene expressed under the control of said transcription factor.

**2-**Use of a compound according to claim 1 for inhibiting the transcription of a given gene under the control of a promotor or enhancer elements which bind to said transcription factor.

**3-**Use of a compound consisting of a double-stranded closed oligonucleotide containing a binding site for transcription factor controlling the expression of a given gene of pathological interest, for preparing a pharmaceutical composition for the therapeutical treatment of pathology in which the said given gene is involved.

**4-**Use of a compound according to any one of claims 1 to 3 wherein said transcription factor is HNF-1 factor.

**5-**Use of a compound according to any one of claims 1 to 4 wherein the said given gene is a liver-specific gene.

**6-**Use of a compound according to claim 5 wherein the said given gene is a gene coding for fibrinogen, transthyretin, apolipoprotein B or pre-S1 gene of hepatitis B virus.

**7-**Use of a compound as in claims 4 to 9 wherein the said compound consists of a double-stranded closed oligonucleotide containing at least the sequence 5'GTTANT 3', and its complementary strand, which is part of the pseudo-palindromic HNF-1 binding sequence.

**8-**Use of a compound according as in claim 4 when the said compound consists of a rouble-stranded closed oligonucleotide containing a binding site for the transcription factor HNF-1 among the followings:

1 5'GTTAATAATCTAC3'
3'CAATTATTAGATG5'

2     5'GTTACTAGTTAAC3'
      3'CAATGATCAATTG5'

3 5'ATTAATAATTACA3'
3'TAATTATTAATGT5'

4 5'GTTAATATTCACC3'
3'CAATTATAAGTGG5'

5    5'GTTAATATTTAAT3'
     3'CAATTATAAATTA5'

6 5'GTTAAAGTATAAC3'
3'CAAT TTCATATTG5'

7 5'GT TACTTAT TCTC3'
3'CAATGAATAAGAG5'

8 5'GTT TATTATTAGC3'
3'CAAATAATAATCG5'

9 5'GAAAATTATTTAC3'
3'CTT TTAATAAATG5'

1 0 5'ATTAATCATTACC3'
3'TAATTAGTAATGG5'

1 1 5'GTAATTGTTTACA3'
3'CATTAACAAATGT5'

1 2 5'GTTAATGTGTAAT3'
3'CAATTACACATTA5'

1 3 5'GTTAAAGTCAGTA3'
3'CAATTTCAGTCAT5'

1 4 5'GTTAATCATTACT3'
3'CAATTAGTAATGA5'

**9-**Use of a compound according to any one of claims 4 to 8 , wherein the closed oligonucleotide is GT-56c represented in table 1.
